Europäisches Patentamt

European Patent Office

Office européen des brevets

(19)

(11) Publication number: 0 373 896
A1

## (12) EUROPEAN PATENT APPLICATION

(21) Application number: 89312993.2

(22) Date of filing: 12.12.89

(51) Int. Cl.5: C12N 9/64, C12N 15/58, A61K 37/547

The microorganism(s) has (have) been deposited with FERM BP 2630 under number(s)

(30) Priority: 12.12.88 JP 314172/88

(43) Date of publication of application:
20.06.90 Bulletin 90/25

(84) Designated Contracting States:
AT BE CH DE ES FR GB GR IT LI LU NL SE

(71) Applicant: YAMANOUCHI PHARMACEUTICAL
CO. LTD.
No. 3-11 Nihonbashi-Honcho, 2-chome
Chuo-ku
Tokyo(JP)

(72) Inventor: Shimizu, Yasuaki
10-15, Takashimadaira 2-chome Itabashi-ku
Tokyo 175(JP)
Inventor: Yano, Emiko
33-8, Takashimadaira 8-chome Itabashi-ku
Tokyo 175(JP)
Inventor: Yano, Shinya

33-8, Takashimadaira 8-chome Itabashi-ku
Tokyo 175(JP)
Inventor: Kato, Masao
257-2, Asukai-cho Higashi-iru Horikawa
Imadekawa
Kamikyo-ku Kyoto-shi Kyoto 602(JP)
Inventor: Kinoshita, Akihito
1-148, Hizen-cho
Takahagi-shi Ibaraki 318(JP)
Inventor: Kawasaki, Tomihisa
18-5, Sengen 1-chome
Tsukuba-shi Ibaraki 305(JP)
Inventor: Ishida, Junko
487-119, Hanakoganei 5-chome
Kodaira-shi Tokyo 187(JP)
Inventor: Gushima, Hiroshi
22-8, Asamadai 3-chome
Ageo-shi Saitama 362(JP)

(74) Representative: Geering, Keith Edwin et al
REDDIE & GROSE 16 Theobalds Road
London WC1X 8PL(GB)

(54) Novel thrombolytic proteins, process for producing the same, and drugs containing the same as active ingredient.

(57) Novel thrombolytic proteins having an amino acid sequence similar to that of natural human tissue plasminogen activator, in which at least one asparagine-linked oligosaccharide chain is attached to a sequence in its epidermal growth factor domain; DNA molecules that code for the amino acid sequences of the novel thrombolytic proteins; and process for producing proteins which comprises introducing the DNA molecule into an expression vector capable of replicating itself in host cells that allow addition of asparagine-linked oligosaccharide chain, transforming said host cells by said expression vector, culturing the transformants thus obtained under conditions that allow expression of said DNA molecule, thereby producing the corresponding protein, and recovering the protein from the culture liquor; and further, agents for the treatment of thrombotic diseases comprising a therapeutically effective amount of the protein and a pharmaceutically acceptable carrier.

# THROMBOLYTIC PROTEINS, THEIR PRODUCTION, CODING DNA MOLECULES THEREFOR, AND DRUGS CONTAINING THE PROTEINS

This invention relates to proteins having an amino acid sequence similar to that of natural human tissue plasminogen activator (natural human t-PA), in which at least one asparagine-binding saccharide chain is attached to a sequence in its epidermal growth factor domain (EGF domain); DNA molecules that code for the amino acid sequences of the novel thrombolytic proteins; a process for producing the proteins; and agents for the treatment of thrombotic diseases comprising a therapeutically effective amount of the protein and a pharmaceutically acceptable carrier. Preferred embodiments of this invention relate to a novel thrombolytic protein having an amino acid sequence similar to that of natural, human t-PA, in which Tyr-67 is replaced by Asn-67 with an asparagine-linked oligosaccharide chain attached thereto; a novel thrombolytic protein having an amino acid sequence similar to that of natural human t-PA, in which Ile-86 is replaced by Asn-86 with an asparagine-linked oligosaccharide chain attached thereto; DNA molecules that code for the amino acid sequences of the above novel thrombolytic proteins; process for producing the above proteins; and agents for the treatment of thrombotic diseases comprising a therapeutically effective amount of above protein and pharmaceutically acceptable carrier.

The proteins of this invention are novel compounds, have an outstandingly prolonged half-life in circulating blood, and show the same clinical effects in a dose markedly smaller than that of natural human t-PA even in a single administration.

This invention relates to novel thrombolytic proteins having physiological activities like those of t-PA. More particularly, it relates to novel, recombinant thrombolytic proteins, to DNA molecules coding for said proteins, to production of said proteins from cells transformed by said DNA molecules, to pharmaceutical compositions containing said proteins, and to the therapeutic use of said proteins as thrombolytic agents.

Herein, amino acids, and amino acids in polypeptides, are abbreviated as listed below according to the method adopted by the IUPAC-IUB Commission on Biochemical Nomenclature ( CBN ).

Ala L-alanine
Arg L-arginine
Asn L-asparagine
Asp L-aspartic acid
Cys L-cystein
Gln L-glutamine
Glu L-glutamic acid
Gly Glycine
His L-histidine
Ile L-isoleucine
Leu L-leucine
Lys L-lysine
Met L-methionine
Phe L-phenylalanine
Pro L-proline
Ser L-serine
Thr L-threonine
Trp L-tryptophan
Tyr L-tyrosine
Val L-valine

The structure of DNA molecules is abbreviated by using the type of base contained in each of the deoxyribonucleotides which constitute the molecule.

A Adenine ( indicating deoxyadenylic acid )
C Cytosine ( indicating deoxycytidylic acid )
G Guanine ( indicating deoxyguanylic acid )
T Thymine ( indicating deoxythymidylic acid )

$(H_2N)$- and -(COOH) show the amino terminus and carboxy terminus of an amino acid sequence, respectively, and $(5')$ and $(3')$ indicate the $5'$-terminal and $3'$-terminal of a DNA sequence, respectively.

Natural human tissue plasminogen activator (natural human t-PA ) is drawing attention as a new thrombolytic agent that will replace streptokinase ( SK ) and urokinase ( UK ) ( Matsuo, "t-PA and Pro-UK", Gakusai-Kikaku, 1987, pp75-176 ).

The sequence of amino acids constituting natural human t-PA and the base sequence of cDNA that codes for the same are already known, both determined by Pennica et al. [ Pennica, D., et al., Nature, 301, 214-221 ( 1983 )]. The numbering system for t-PA amino acid sequence in this specification is in accordance with the system adopted in this article of Pennica et al., while the base sequence is numbered with the A of ATG ( translation initiation codon ) in t-PA taken as the start point.

t-PA is first synthesized in vascular endothelial cells as a precursor containing prepro-sequence ( composed of 562 amino acid residues ), which is then cleaved at the site between Arg-(-1) and Ser-1 by the action of an intracellular enzyme, and the resulting single-stranded glycoprotein composed of 527 amino acid residues is secreted outside the cells. This single-stranded t-PA further undergoes cleavage by the action of plasmin at the site between Arg-275 and Ile-276, turning into a double-stranded structure consisting of H(A)-chain with N-terminal and L(B)-chain with C-terminal. The H-chain is presumed to be constructed of four structural domains because of homology with other proteins in terms of amino acid sequence [ Ny, T. et al., Proc. Natl. Acad. Sci. USA, 81, 5355-5359 ( 1984 ); Patthy, L., Cell, 41, 657-663 ( 1985 )]. The four domains are named, from the N-terminal side, finger (F), epidermal growth factor (EPG), kringle 1 (K1), and kringle 2 ( K2 ) domains. The two kringle domains are highly homologous with each other ( as high as about 55 % homology ). F and K2 domains are considered to participate in promoting protealytic activity against proteins having affinity for fibrin and fibrin-dependent proteins [ Verheijen, J. H., et al., EMBO J., 5, 3525-3530 ( 1986 ); van Zonneveld, A-J., et al., J. Biol. Chem., 261, 14214-14218 ( 1986 )]. EGF domain is a hydrophobic domain, which is known to play no important role in the fibrinolytic activity of t-PA. The L-chain, on the other hand, has protealytic activity and shows high homology with various serine proteases.

Natural human t-PA is a glycoprotein which contains, in the molecule, three amino acid sequences of Asn-X-Thr or Asn-X-Ser ( X is a naturally occurring amino acid other than Pro ) to which an asparagine-linked oligosaccharide chain can be attached. In fact, oligosaccharide chains are attached to Asn-117, Asn-184 and Asn-448 in t-PA, but molecular species in which no oligosaccharide chain is attached to Asn-184 are also known. The molecular species of t-PA with oligosaccharide chains attached to three asparagine residues ( 117, 184 and 448 ) is generally called type I, while the species with oligosaccharide chains attached only to two asparagine residues ( 117 and 448 ) is called type II. The difference in molecular weight between the two types is about 2000 to 4000 daltons.

The most notable feature of natural human t-PA is its high affinity for fibrin. It is regarded as a thrombolytic agent with less side effects, such as bleeding because it activates plasminogen bound to fibrin very effectively in contrast to SK and UK having no affinity for fibrin, although it does not effectively activate plasminogen in circulating blood. Actual clinical tests demonstrated that intravenously administered natural human t-PA is effective in causing re-perfusion of the obstructive coronary artery in patients with myocardial infarction [ The TIMI Study Group, N. Engl. J. Med., 312, 932-936 ( 1985 ); European Cooperative Study Group for Recombinant t-PA, Lancet, 1, 842-847 ( 1985 )].

However, the problem involved in the use of human t-PA for the treatment of thrombotic diseases is the extremely short half-life of its enzyme activity in circulating blood (three to six minutes ) [ Verstraete, M. et al., J. Pharmacol. Exp. Ther., 235, 506-512 ( 1985 ); Verstraete, M. et al., Thromb. Haemost., 56, 1-5 ( 1986 ); Nguyen, G. et al., Thromb. Haemost., 58, 437 ( 1987 ) ( Abstract )]. Rapid clearance of t-PA in vivo is due mainly to the liver, and it is suggested that t-PA is recognized by the liver mainly through its H-chain [ Rijken, D. C. and Emeis, J. J., Biochem. J., 238, 643-646 ( 1986 )]. Hence, when used for the treatment of thrombotic diseases, natural human t-PA must be continuously administered intravenously in a high dose ( 50 to 100 mg ) in order to maintain its concentration in the blood at a sufficiently high level. As a result, treatment by the use of human t-PA is very costly and can cause exacerbation of systemic fibrinolysis and bleeding accompanied by high-dose administration.

Under the circumstances, there has been a demand for the development of a new type of t-PA having a prolonged half-life in the circulation and retaining the affinity for fibrin and fibrin dependency.

Intensive studies to develop a new type of t-PA by recombinant DNA technology have led us to find that new thrombolytic proteins having an outstandingly prolonged half-life in the circulation and retaining the desirable physiological properties of human t-PA can be obtained if an amino acid sequence with a saccharide chain attached thereto is disposed in the EGF domain of natural human t-PA.

This invention provides novel thrombolytic proteins having an amino acid sequence similar to that of natural human t-PA, in which at least one asparagine-linked oligosaccharide chain is attached to a sequence in its EGF domain. EGF domain herein means a domain from about Val-48 to about Thr-91 in the amino acid sequence of natural human t-PA. The amino acid sequence with an asparagine-linked oligosaccharide chain attached thereto is a sequence of Asn-X-Thr or Asn-X-Ser ( in which X is an amino acid other than Pro, and the saccharide chain is attached to Asn ). Proteins of this invention containing such amino acid sequence include modifications of natural, human t-PA in which part of the amino acids in the EGF domain

3

are replaced by other amino acids, those in which other amino acids are inserted in the EGF domain, those in which part of the amino acids in the EGF domain are deleted, and those in which two or more of the above three types of alterations ( replacement, insertion and deletion of amino acid ) are made in the EGF domain. Shown below are illustrative examples of these modifications.

Examples of replacement are as follows: Asn-48 in place of Val-48, Asn-50 in place of Ser-50, Ser-60 or Thr-60 in place of Gly-60, Asn-59 in place of Gly-59, Asn-67 in place of Tyr-67, Asn-86 in place of Ile-86, and Asn-89 in place of Arg-89. Such replacement may be made at two or more sites in the EGF domain. Of these, replacement of Tyr-67 by Asn-67 and replacement of Ile-86 by Asn-86 are the most preferred.

Typical examples of insertion are as follows: insertion of Asn between Val-48 and Lys-49, insertion of Ser, Thr or Asn between Gly-59 and Gly-60, insertion of Asn between Tyr-67 and Phe-68, insertion of Asn between Ile-86 and Asp-87, and insertion of Asn between Arg-89 and Ala-90. Such insertion may be made at two or more sites in the EGF domain.

Examples of deletion are those devoid of Gly-59 or Gly-60.

In addition to the illustrative examples shown above, the proteins of this invention may also include those obtained by replacement, insertion or deletion of two or more amino acids, and by combinations thereof.

The proteins of this invention contain at least one asparagine-linked oligosaccharide chain attached to the modified amino acid sequence in the EGF domain.

Described below is a process for producing thrombolytic proteins of this invention.

(a) Preparation of DNA molecule that codes for human t-PA

Thrombolytic proteins of this invention may be produced by various recombinant DNA techniques using cDNA clone for human t-PA as the starting material. cDNA clone that codes for human t-PA precursor (human t-PA cDNA) can be obtained by preparing a cDNA bank from mRNA derived from Bowes human melanoma cells by the known method, followed by isolation from said cDNA bank using the colony hybridization technique ( refer to Example 1 ).

The expression vector for animal cells ( pVY1A ), used for expression of natural human t-PA and thrombolytic proteins of this invention, allows insertion of foreign gene at its restriction enzyme site of BglII. On the other hand, cDNA for human t-PA has no BglII site upstream of the N-terminal of the prepro-sequence, although this cleavage site is present at the junction of prepro-sequence and mature t-PA ( between Arg-(-1) and Ser-1 ). Hence, cDNA for t-PA should preferably be modified so that it may be easily inserted at the BglII site in pVY1A. To this end, it is possible to delete the BglII site between Arg-(-1) and Ser-1 by changing the codon coding for Arg from AGA to CGA, and to provide a new BglII site 5′-upstream of initiation codon ( ATG ) at the N-terminal of the prepro-sequence. In addition, the 3′ non-translation region which is superfluous should preferably be deleted by digestion with Xho II. The modified cDNA for t-PA thus prepared is then converted into a starting material for the production of novel thrombolytic protein by inserting it into a phagemid (e.g., pTZ18/19 and pUC118/119) or a phage DNA (e.g., M13mp18/mp19) which can be used for site-specific mutagenesis.

Because the amino acid sequence of t-PA is known as described above, the above modified cDNA for t-PA can be chemically synthesized by selecting proper codons coding for amino acids - by conceptionally dividing the DNA corresponding to t-pA precursor into several oligonucleotides, chemically synthesizing these oligonucleotides, and ligating these by the usual method. Each of the oligonucleotides is preferably prepared by the phosphite method using a commercial fully-automatic DNA synthesizer.

In addition modified t-PA DNA can be prepared by replacing prepro-sequence of t-PA precursor with other prepro-sequence having the highest production efficiency in relation with host cells selected for expression.

(b) Preparation of DNA molecules that code for novel thrombolytic proteins

The DNA molecule that codes for a novel thrombolytic protein can be prepared by partially modifying the DNA sequence in a specific domain of t-PA DNA by the conventional site-specific mutagenesis technique. This partial modification is effected by replacement, deletion or insertion of at least one amino acid in the EGF domain, or by a combination thereof, so as to give a base sequence that codes for an amino acid sequence comprising Asn-X-Ser or Asn-X-Thr (X is a naturally occurring amino acid other than Pro) in the EGF domain of natural human t-PA.

4

(c) Production of novel thrombolytic proteins

A DNA molecule of this invention codes for a novel thrombolytic protein. Hence, its expression in host cells that allow addition of an asparagine-linked oligosaccharide chain enables production of a novel thrombolytic protein having an amino acid sequence with at least one asparagine-linked oligosaccharide chain attached thereto in its EGF domain. Suitable mammalian host cells, expression vectors for the same, methods of transformation, methods of cultivation and methods of purifying final products are all known in this field. These are disclosed, for example, in Haynes, J. and Weissmann, C., Nucl. Acids Res., 11, 687-706 ( 1983 ); Bebbingston, C.R. and Hentschel, C.C.G., In Glover, D.M. (ed), DNA-Cloning Vol. III, IRL Press, Oxford ( 1987 ), pp163-188; Freshney, R.I., Culture of Animal Cells, Alan R. Liss, Inc. NY ( 1987 ); Gorman, C., In Glover, D.M. (ed), DNA Cloning Vol. II, IRL Press, Oxford ( 1985 ), pp143-190; Perbal, B.A., Practical Guide to Molecular Clnoing, John Wiley & Sons, Inc. ( 1984 ), pp487-543; Japanese Patent Publication No.16931/1987 ( published on April 15, 1987 ); and The Biochemical Society of Japan (ed), Biochemical Experiment Courses ( continued ), Course 8, Blood ( second ), Tokyo Kagaku Dozin ( 1987 ), pp603-610.

In Examples 6 to 7, DNA molecule that codes for a novel thrombolytic protein was inserted into an expression vector pVY1A, which is composed of a BamHI-KpnI fragment ( about 2900 base pairs ) of plasmid pKSV10 ( Pharmacia Fine Chemicals ) and plasmid pAdD26SV(A) No.3 ( supplied by Dr. Hiroshi Handa of Tokyo University ). The above BamHI-KpnI fragment contains transcribed unit of SV40 early gene ( including SV40 early promoter, intervening sequence, poly(A) addition signal, etc. ), and the DNA molecule of this invention can be expressed by utilizing this transcription unit. pAdD26SV(A) No.3 codes for mouse dihydrofolate reductase ( DHFR ) under the control of major late promoter of adenovirus type-2 and is capable of intracellular amplificator using methotolexate. Other mammalian cells that can be used for practicing this invention include VERO cells, CV-1 cells, LCC-MK$_2$ cells, COS-1 and COS-7 cells, HeLa cells, 293 cells, RPMI8226 cells, 3T3 cells, BHK cells, WI38 cells and CH0-K1 cells. The DNA molecules of this invention can also be expressed under the control of SV40 late gene or promoter derived from t-PA or actin, or a promoter derived from viral genes, such as adenovirus major late gene, cytomegalovirus immediate early gene, and LTR ( long terminal repeat ) of various retroviruses.

The novel thrombolytic proteins of this invention can be produced in the cells of insects and yeast. Useful host cells, expression vectors, methods of trans formation, methods of cultivation and methods of purifying final products are all known in this field. These are disclosed in, for example, Maeda, S., Virus, 37, 1-11 ( 1986 ); Nakao, J., Experimental Medicine, 5, 136-141 ( 1987 ); Carter, B.L.A., et al., In Glover, D.M. (ed), DNA Cloning, Vol. III, IRL Press, Oxford ( 1987 ), pp141-161; Chigami Y. and Tanaka H., Protein Engineering Laboratory Manual ( edited by Ikehara M. and Miki K. ); and Kodansha Scientific ( 1988 ), pp98-120.

It is known that t-PAs produced and secreted by animal cells have various N-terminal sequences depending on the difference in N-terminal cleavage site. As generally known N-terminal sequences may be mentioned the sequence starting with Ser-1, the one with the three amino acids at the N-terminal ( Ser Tyr Gln ) deleted and starting with Val-4, and the one in which three amino acids ( Gly Ala Arg ) are disposed upstream of Ser-1. When using animal cells as the host, as described above, t-PAs with different N-terminals can be produced because processing by the action of signal peptidase or protease differs depending on the type of cell used. This phenomenon occurs not only in secretion and production by animal cells; a similar phenomenon also occurs in the modification observed at the N-terminal of t-PA produced by yeast and other culture cells. The thrombolytic proteins having any type of N-terminal sequences described above are all included in this invention.

It is also known that, when human t-PA gene was expressed in culture cells, the number and chemical structure of oligosaccharide chains attached to t-PA differ depending on the type of host cells used. The proteins obtained by the expression of DNA molecues of this invention are all included in this invention regardless of the type and number of oligosaccharide chains.

The novel proteins thus produced are recovered and purified by known techniques, and subjected to physico-chemical, biochemical and clinical tests.

The novel thrombolytic proteins of this invention thus obtained are particularly useful for the treatment of acute myocardial infarction like natural human t-PA. It has recently been provided that natural human t-PA, when intravenously administered in a dose of 50 to 100 mg over a period of one to three hours, is effective in dissolving occlusive coronary arterial thrombi, regenerating myocardiac perfusion and restoring most of the ischemmic myocardiac layer. The novel proteins of this invention, which have an outstandingly prolonged half-life in the circulation, are expected to show the same clinical effects as above in a dose markedly smaller than that of natural human t-PA even in a single administration as a bolus.

The novel proteins of this invention are also effective in treating a great variety of acquired diseases involving vascular coagulation, such as deep vein thrombosis, pulmonary arterial embolism, peripheral arterial thrombosis, heart or peripheral artery-derived embolism, acute myocardial infarction and thrombotic attacks.

The following examples will further illustrate the invention but are not intended to limit its scope. In practicing this invention, DNA recombination operations were performed, unless otherwise specified, according to the procedures described in the following book.

Maniatis, T., et al., Molecular Cloning: A Laboratory Manual, Cold Spring Harbor Laboratory, Cold Spring Harbor, NY ( 1982)

DNA digestion with various restriction enzymes was carried out, unless otherwise specified, according to the operating manuals for respective enzymes. Synthetic oligonucleotides were all prepared by the β-cyanophosphamidide method using a Model-380A DNA Synthesizer ( Applied Biosystems ). Synthesis of DNA oligomers, removal of protective groups, cleavage from resin and purification were carried out according to the Manual for the Model-380A DNA Synthesizer.

Example 1 Cloning of cDNA for t-PA

Bowes melanoma cells ( supplied by Dr. Roblin, R. of the National Cancer Institue, USA) were grown according to the method of Opdenakker et. al [ Opdenakker, G. et al., J. Biochem., 131, 481-487 ( 1983 )]. In order to induce mRNA for t-PA, TPA ( 12-O-tetradecanoylphorbol 13-acetate ) was added to the culture mixture to a final concentration of 100 ng/ml, and cultivation was further continued for 16 hours. The total cellular RNA was then extracted from the culture cells according to the modified method of Freeman et. al [ Okayama/Berg, cDNA Manual, page 3 ( 1985 ), Pharmacia Fine Chemicals )], and poly(A)$^+$ RNA was isolated from the total cellular RNA using an oligo-dT cellulose column ( Pharmacia ), giving approximately 400 μg of poly(A)$^+$ from about $10^9$ cells.

This poly(A)$^+$ RNA was fractionated by sucrose density gradient centrifugation in usual manner, and part of each fraction was subjected to dot blot hybridization using an oligonucleotide probe specific to mRNA for t-PA [ Pebal, B., A Practical Guide to Molecular Cloning, page 410 ( 1984 ), John Wiley & Sons Inc. ] to detect the fraction of mRNA for t-PA. The probe used in this case ( probe Y ) has a base sequence of 5′-GCTTGGCAAAGATGGCA-3′ which is complementary to the mRNA region encoding the 291-297 amino acid sequence of t-PA reported by Pennica et al. Proble Y was labelled at its 5′-terminal using T4 polynucleotide kinase ( Takara Shuzo Co., Ltd. ) and γ-[$^{32}$P]ATP according to the Laboratory Manual ( page 122 ).

Probe Y was strongly hybridized mainly with 20 to 30S poly(A)$^+$ RNA ( hereinafter referred to as fraction M ).

Poly(A)$^+$ RNA obtained from fraction M ( 10 μg ) was used as a template to give 3 μg of double-stranded cDNA by the action of reverse transcriptase ( Biochemical Industry Co., Ltd. ) according to Gubler-Hoffman's method [ Gubler, U. and Hoffman, B.J., Gene, 25, 263 ( 1983 )], and deoxy C chain was attached to the double-stranded cDNA at its 3′-terminal according to Deng-Wu's method [ Deng, G.R. and Wu, R., Nucleic Acid Res., 9, 4173 ( 1981 )]. The product thus obtained was then subjected to gel filtration through CL4B Sepharose (Pharmacia) to remove low- molecular weight nucleic acids of less than about 500 bp, followed by annealing with pBR322 having deoxy G chain attached at its Pst I site ( Bethesda Research Lab. ) by the usual method. The resulting mixture was used to transform E. coli HB101 competent cells ( Takara Shuzo ), giving a cDNA bank composed of about 40,000 independent transformants.

This cDNA bank was subjected to colony hybridization using the above-mentioned probe Y according to Woods' method [ Woods, D., Focus, 6(3), 1 ( 1984 ); Bethesda Research Lab. ] to obtain clones reacted with probe Y. From these clones, was selected the one containing the longest t-PA cDNA ( about 2500 bp ), and the base sequence of its plasmid pBR322tPA ( #42 ) was determined by the dideoxy method using M13 phage vector [ Carlson, J. et al., J. Biotechnology, 1, 253 ( 1984 )] and by the 7-DEAZA method [ Mizusawa, S. et al., Nucleic Acids Research., 14, 1319 ( 1986 )]. It was demonstrated that plasmid pBR322tPA ( #42 ) contains the whole base sequence of gene that codes for t-PA precursor.

Example 2 Construction of pTZ18tPA002

A) Construction of pTZ18tPA002

6

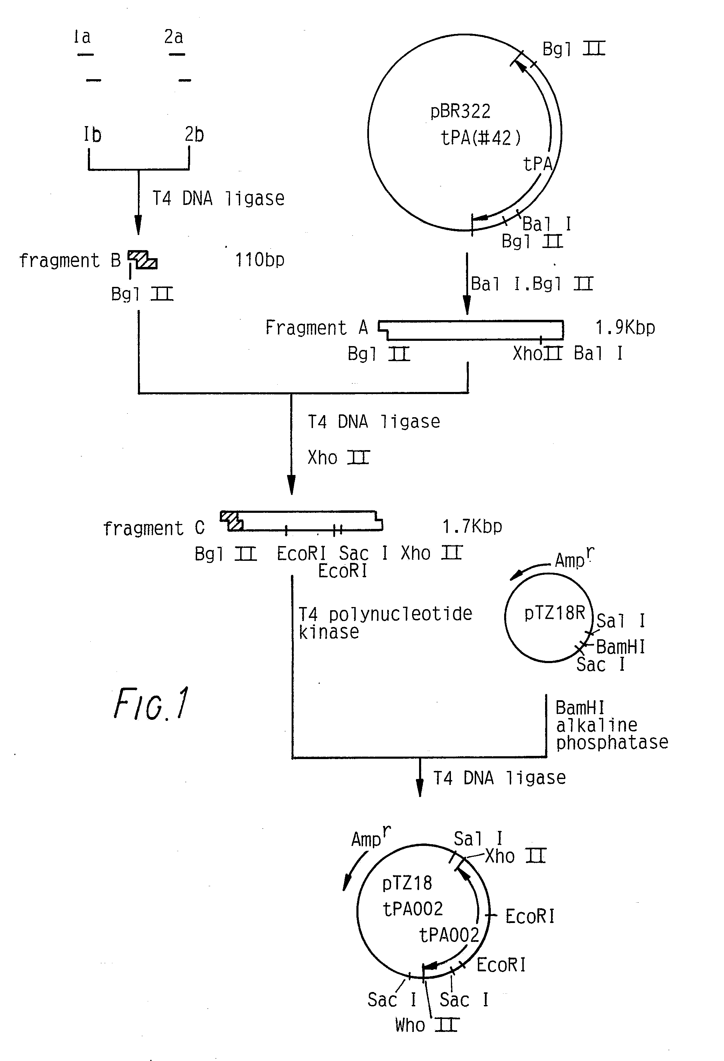

In order to introduce the gene that codes for t-PA precursor into expression vector pVY1A at the Bgl II site, we constructed a modified t-PA gene ( tPA002 gene ), in which the Bgl II site in nucleotide 103 is deleted and a new Bgl II site is provided immediately before the translation start codon ( ATG ), as shown in Figure 1.

Plasmid pBR322tPA ( #42 ) was digested with Bal I ( Takara Shuzo ) and with Bgl II ( Takara Shuzo ) in that order; the digests were subjected to electrophoresis on 1.0% agarose gel, and the Bgl II-Bal I fragment ( fragment A)of about 1.9 Kbp derived from t-PA cDNA was cut out from the gel. Fragment A was purified by electro- elution using a DNA Cell (Trade Name, Daiichi Chemicals Co., Ltd. ). Extraction of the final product was carried out according to the Operation Manual of the DNA Cell.

Next, the four oligonucleotides ( 1a, 1b, 2a and 2b ) shown below were synthesized.

```
                1 a
5′ GAT CTA TGG ATG CAA TGA AGA GAG
   GGC TCT GCT GTG TGC TGC TGC TGT
   GTG GAG C   3′
                1 b
5′ AAG ACT GCT CCA CAC AGC AGC AGC
   ACA CAG CAG AGC CCT CTC TTC ATT
   GCA TCC ATA   3′
                2 a
5′ AGT CTT CGT TTC GCC CAG CCA GGA
   AAT CCA TGC CCG ATT CAG AAG AGG
   CGC CC  3′
                2 b
5′ GAT CGG GCG CCT CTT CTG AAT CGG
   GCA TGG ATT TCC TGG CTG GGC GAA
   ACG   3′
```

After purification by HPLC, 100 pmol of oligonucleotide 1b was mixed with 10 units of T4 polynucleotide kinase in 50 μl of kinase buffer [ 50mM Tris-HCl ( pH 8.0 ),10mM MgCl2, 5mM DTT and 1mM ATP), and the mixture was incubated at 37ºC for 90 minutes to effect phosphorylation, followed by treatment with phenol/chloroform. Oligonucleotide 2a was treated in the same way, the phosporylated oligonucleotides 1b and 2a thus obtained were mixed together, oligonucleotides 1a and 2b ( 100 pmol each ) and tRNA ( 7.8 μg ) were added to this mixture, and ethanol was then added to effect precipitation. The precipitate formed was collected by centrifugation and dissolved in 43 μl sterile water, the solution was allowed to cool slowly from 75ºC, 5 μl of T4 DNA ligase buffer of 10 times higher concentration [ 66mM Tris-HCl ( pH 7.6 ), 6.6mM MgCl2, 10mM DTT and 0.4mM ATP ) and 700 units of T4 DNA ligase ( Takara Shuzo ) were added to the above cooled solution, and the resulting mixture was incubated overnight at 16ºC, giving a fragment of about 110 bp ( fragment B ).

The fragment A derived from t-PA gene, the fragment B obtained above and 350 units of T4 DNA ligase were mixed together in T4 DNA ligase buffer, the mixture was incubated overnight at 16ºC to effect ligation, and the reaction mixture was heated at 75ºC for 15 minutes and then digested with Xho II ( Boeringer Mannheim ). Fraction B, having two sticky ends, can be linked to fraction A in two different ways. When oligonucleotides 2a and 2b were linked with fragment A, the formed linkages were not cleaved with Xho II because the base sequences of these oligonucleotides had been modified so as not to regenerate the original Bgl II ( or Xho II) sites. On the other hand, when oligonucleotides 1a and 1b were linked with

7

fragment A, the linkages formed were cleaved with Xho II. Fragment of about 1.7 kbp was cut out from the gel and purified by electro - elution.

The DNA fragment thus obtained was composed mainly of tPA002 gene fragment of about 1.7 kbp formed by linkage between fragments A and B followed by digestion with Xho II ( fragment C ), but it also contained a small amount of a different fragment of about 1.6 kbp formed by digestion of fragment A with Xho II ( fragment D ).

This tPA002 gene fragment was then inserted into the BamH I site of pTZ18R to form single-stranded t-PA gene as shown in Figure 1. A mixture of fragments C and D was subjected to phosphorylation by the action of T4 polynucleotide kinase, and the reaction mixture was heated at 75°C for 15 minutes. Separately, 6.25 μg of pTZ18R ( Pharmacia ) was digested with BamH I (Takara Shuzo), followed by treatment with phenol/chloroform and precipitation with ethanol, the precipitate thus formed was mixed with 0.13 unit of alkaline phosphatase ( Takara Shuzo ) in 0.9 M Tris-HCl ( pH 8.0 ), and the resulting mixture was incubated at 65°C for one hour, followed by treatment with phenol/chloroform and precipitation by addition of ethanol.

This treated pTZ18R ( 1 μg ) was then linked with phosphorylated fragments C and D (1 μg ) by the action of 350 units T4 DNA ligase, and the reaction mixture was used to transform E. coli NM522 competent cells ( Funakoshi ) according to Hanahan's method [ Hanahan, D., J. Mol. Biol., 166, 57 ( 1983 )]. The transformants thus prepared were grown overnight at 37°C on an ampicillin-containing LB plate ( 10 g bactotrypton, 5 g yeast extract, 10 g NaCl, 15 g/l bactoagar, 0.01M MgSO₄, 0.1 mM IPTG, 0.004% X-gal ), the white colonies developed were then cultured in 2 ml of ampicillin-containing 2 x YT ( 16 g bactotrypton, 10 g yeast extract and 5 g/l NaCl ), and plasmid DNA was isolated by the rapid method ( alkalilysis method ) according to Laboratory Manual ( page 368-369 ).

Next, a plasmid showing bands of about 2.85 kbp, about 740 bp, about 550 bp and about 470 bp ( containing fragment C ) was isolated after double digestion with Sal I ( Takara Shuzo ) and EcoR I ( Takara Shuzo ). Fragment C was inserted into pTZ18R in two different directions, and the plasmid showing two bands of about 4.2 kbp and about 410 bp after digestion with Sac I ( Takara Shuzo ) ( pTZ18tPA002 ) was used in the following experiments. Correct linkage of synthetic fraction B with fraction A was demonstrated by determination of the base sequence of the resultant plasmid pTZ18tPA002 using M13 primer M4 (Takara Shuzo), which was carried out according to M13/pUC Sequencing Manual (Nippon Gene Co.,Ltd.; 1986, page 19-20).

B) Preparation of single-stranded pTZ18tPA002

A single-stranded DNA to be used for site-specific mutation was first prepared from pTZ18tPA002 transformant as described below.

pTZ18tPA002 transformant was grown overnight in 2 ml of 2 x YT containing ampicillin and 0.01% thiamin. 150 μl of the culture solution thus obtained was added to 15 ml of 2 x YT containing ampicillin and thiamin, and the mixture was incubated at 37°C for one hour. It was then infected with 250 μl of 2.5 x 10⁹ pfu/ml helper phage M13K07 ( Takara Shuzo ), 42 μl of kanamycin ( 25 mg/ml ) was further added, and cultivation was further continued overnight. The culture solution was centrifuged at 0°C for five minutes at 12,000 rpm, the supernatant was transferred to another centrifuge tube, 3 ml of an aqueous solution containing 20% PEG and 2.5M NaCl was added, and the mixture was allowed to stand at room temperature for 20 minutes and again centrifuged at 20°C for 10 minutes at 12,000 rpm. The precipitate thus obtained was dissolved in TE buffer ( pH 8.0 ), the solution was treated with phenol, the mixture was centrifuged, and the aqueous layer was transferred to another centrifuge tube and subjected to ethanol precipitation, giving 7.6 μg of single-stranded pTZ18tPA002.

Example 3 Construction of expression vector pVY1A

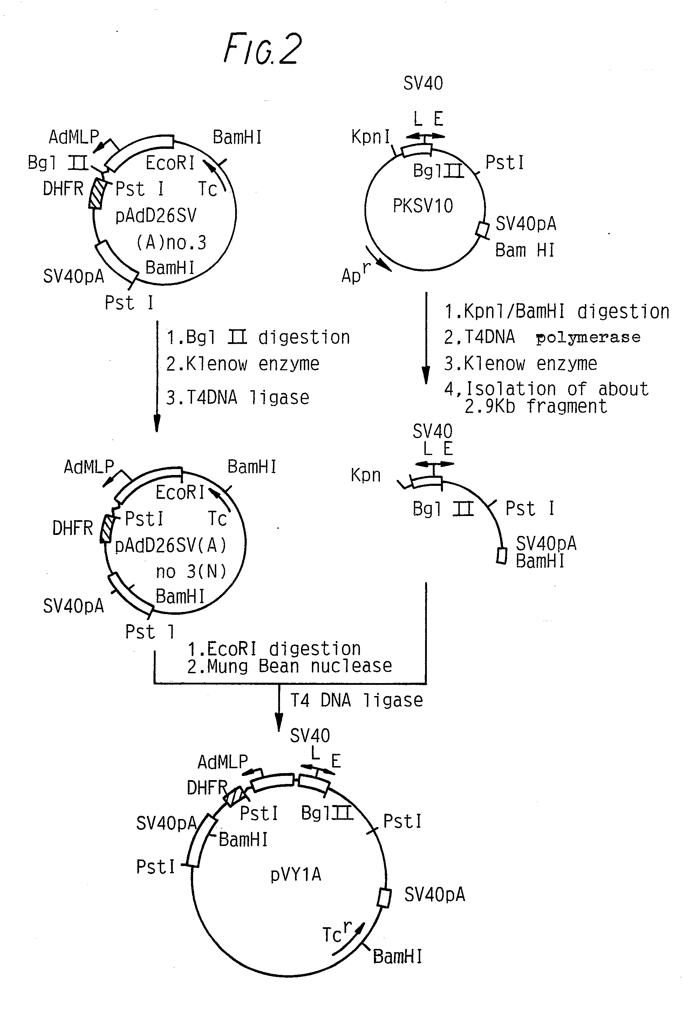

pVY1A was constructed as shown in Figure 2.

A) Construction of pAdD26SV(A) No.3(N) and its digestion with EcoR I

Vector pAdD26SV(A) No.3(N) [ supplied by Dr. Hiroshi Handa of Tokyo University; already disclosed in Kaufman, R.J. and Sharp, P.A., Mol. Cell. Biol., 2, 1304-1319 ( 1982 )] was cleaved with Bgl II, the reaction mixture was extracted with phenol-chloroform, the plasmid DNA then precipitated with ethanol, and the precipitate thus obtained dissolved in sterile distilled water. After making the cleaved ends blunt by the use of Klenow enzyme ( Boeringer Mannheim ), the solution was treated with phenol-chloroform, the DNA was precipitated with ethanol and the precipitate dissolved in sterile distilled water. After self-ligation using a DNA ligation kit (Takara Shuzo), the resulting reaction mixture (plasmid) was used to transform E. coli HB101 competent cells. From the transformants resistant to tetracycline were isolated plasmid DNAs, which were cleaved with Bgl II and subjected to electrophoresis on 0.7% agarose gel, giving cloned pAdD26SV(A) No.3(N) with the Bgl II site deleted.

This plasmid DNA was then digested with EcoR I, treated with phenol/chloroform and precipitated by addition of ethanol. The precipitate thus formed was dissolved in sterile distilled water, and the EcoR I cleaved ends were made blunt by the use of Mung-bean nuclease ( Pharmacia ). After treatment with phenol-CHCl3 and ethanol precipitation, the precipitate thus formed was dissolved in sterile distilled water.

B) Isolation of Kpn I-BamH I fragment ( about 2.9 Kbp ) from pKSV10

pKSV10 DNA ( Pharmacia ) was cleaved with Kpn I and BamH I in usual manner, the cleaved ends were made blunt by the use of T4 DNA polymerase ( Takara Shuzo ) and Klenow enzyme according to Laboratory Manual ( page 394-3995 ), and a fragment of about 2.9 Kbp was isolated by electrophoresis on 0.7% agarose gel and recovered by electro-elution.

C) Construction of pVY1A

The DNA fragments obtained in A) and B) above were linked together using DNA ligation kit, and the ligated product was used to transform E. coli HB101 competent cells.

From the transformants resistant to tetracycline were prepared plasmid DNAs by the usual method; these DNAs were digested with Pst I ( Boeringer Mannheim ), followed by electrophoresis on 1.0% agarose gel, giving plasmid pVY1A showing bands of about 3.6 Kbp, about 3.25 Kbp and about 1.5 Kbp. This clone ( E. coli HB101-pVY1A ) was deposited in the Fermentation Reseasrch Institute under the Agency of Industrial Science and Technology of Japan under Registration No. 2630 (FERM BP-2630) on 24th November 1988.

Example 4 Synthesis of primers for inducing site-specific mutation

Single-stranded pTZ18tPA002 obtained in Example 2 B) was subjected to site-specific mutation, preparing tPA023 gene that codes for tPA023 ( natural human t-PA with its Tyr-67 replaced by Asn ). Primer 3 was first synthesized for inducing mutation and screening, and primer 4 was synthesized for determining the base sequence in the mutated region.

```
                                                    67

                                    Gln Ala Leu Tyr Phe Ser

Sequence of natural,            5'  AG GCC CTG TAC TTC TCA 3'
human t-PA
                                                    Asn

                                5'  AG GCC CTG AAC TTC TCA 3'

Primer 3                        3'  TC CGG GAC TTG AAG AGT 5'
( for inducing mutation )

Primer 4                        5' TGG TCC TCG TAG CAC GT  3'
( for determining base sequence )
```

The amino acid sequence of natural human t-PA and nucleotide sequence of the reading strand are given in the upper two rows. The squences of primers 3 and 4 are represented by opposite strands.

Example 5 Inducing site-specific mutation

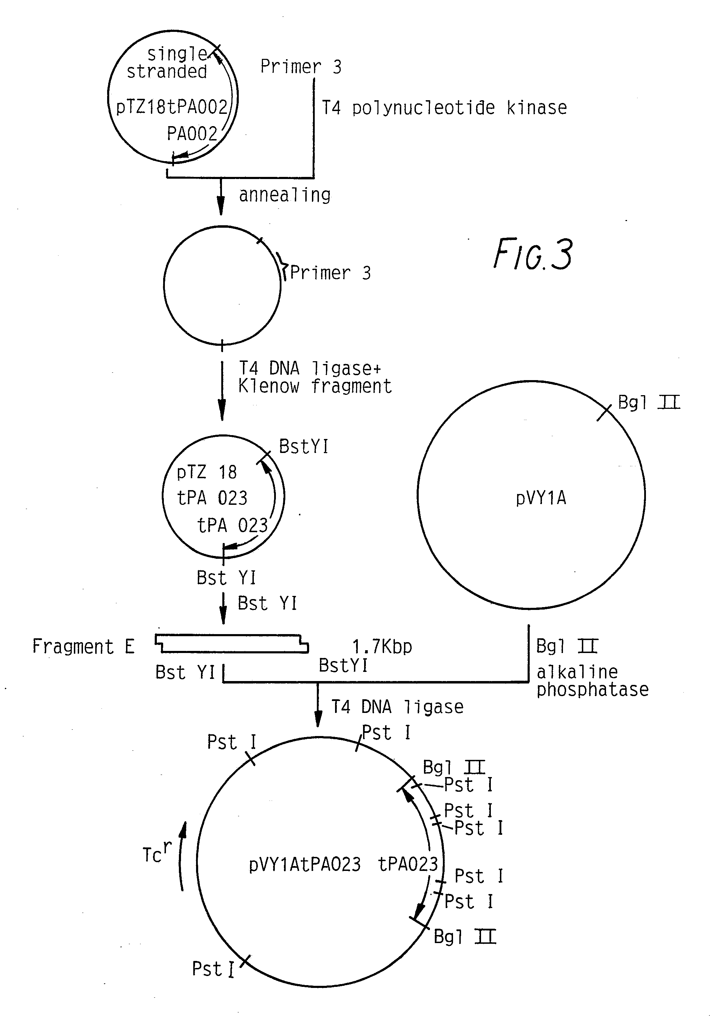

The method of constructing pTZ18tPA023 is shown in Figure 3.

Site-specific mutants were prepared according to the method of Gillam et al. [ Gillams, S., Zoller, M. and Smith, M.: Mutant Construction by in-vitro Mutagenesis, in Dillon, J.A.R., Nasim, A. and Nestman, E.R. (Eds), Recombinant DNA Methodology, John Wiley & Sons ( 1985 ), pp.157-186 ], and colony hybridization was performed by the method of Woods described in Example 1.

Synthetic primer 3 shown in Example 4 was phosphorylated using T4 polynucleotide kinase, the phosphorylated primer ( 4 pmol) was mixed with single-stranded pTZ18tPA002 ( 0.2 pmol ) in 10 $\mu$l of Klenow buffer [ 50 mM Tris-HCl ( pH 7.5 ), 10mM $MgCl_2$, 50mM NaCl, 1mM DTT ), the mixture was incubated at 100°C for one minute, then cooled in ice and again incubated at 40°C for 30 minutes, and 10 $\mu$l of a ligation solution [ 20mM Tris-HCl ( pH 7.5 ), 10mM $MgCl_2$, 10mM DTT, 1mM each of dATP, dGTP, dCTP and dTTP, 1mM ATP, 525 units of T4 DNA ligase ] and 3.5 units of Klenow fragment ( Takara Shuzo ) were added to start primer extension. After incubation overnight at 15°C, the reaction mixture was used to transform E. coli MV1304 competent cells, and the transformants were grown overnight on an ampicillin-containing LB-agar plate at 37°C. The colonies thus formed were transferred onto a sheet of nitrocellulose filter, which was treated successively with 0.5M NaOH solution for ten minutes, with 1.0M Tris-HCl solution (pH 7.4) for five minutes and with 1.5M NaCl-1.0M Tris-HCl solution ( pH 7.4 ) for five minutes, and air-dried. The dry filter was then heated at 75°C for 90 minutes in vaccum, and incubated together with a hybridization solution ( 6 x SSC, 5 x Denhardt's solution, 0.2% SDS, 0.05% sodium pyrophosphate, 100 $\mu$g/ml Salmon sperm DNA ) at 54°C for two hours. Primer 3 with its 5′-terminal labelled with $\gamma$-$^{32}$P was then added as probe to the filter, which was held at 46°C overnight to effect hybridization 1 x SSC is a solution containing 0.15M NaCl and 0.015M trisodium citrate ( pH 7.0 ), while 1 x Denhardt's solution contains 0.02% Ficoll, 0.02% polyvinylpyrrolidone and 0.02% BSA. The thus treated filter was washed with 6 x SSC 0.05% sodium pyrophosphate solution twice at room temperature, twice at 44°C and once at 49.5°C, air-dried, and submitted to autoradiography. Each positive colony was cultured, and plasmid DNA was isolated by the rapid isolation method and incubated overnight at 37°C in 50 $\mu$l of 40 $\mu$g/ml RMase solution.

To the reaction mixture thus obtained ( 50 $\mu$l ) was added 30 $\mu$l of 20% PEG-2.5M NaCl solution, the resulting mixture was allowed to stand overnight at 4°C and centrifuged, and the separated precipitate was collected, washed with 75% ethanol and dried. Base sequence was determined by the dideoxy method using primer 4 shown in Example 4, and plasmid pTZ18tPA023, in which base sequence TAC corresponding to Tyr-67 in t-PA had been mutated to AAC corresponding to Asn, was picked out.

This plasmid was digested with BstY I ( New England Biolabs. ), the reaction mixture was subjected to electrophoresis on 1.0% agarose gel, and tPA 023 gene fragment of about 1.7 Kbp ( fragment E ) was isolated by electro-elution.

Example 6 Subcloning of tPA023 gene in expression vector pVY1A

tPA023 gene was integrated into expression vector pVY1A as shown in figure 3.

Expression vector pVY1A was digested with Bgl II, the reaction mixture was treated with phenol-$CHCl_3$, and ethanol was added to effect precipitation. The DNA thus obtained was subjected to treatment with alkaline phosphatase as described in Example 2, followed by treatment with phenol-$CHCl_3$ and ethanol precipitation, and this phosphatase - treated pVY1A was linked with fragment E using T4 DNA ligase.

The resulting reaction mixture was used to transform E. coli HB101 competent cells, and the transformants were grown overnight on LB agar medium containing tetracycline. The developed colonies were cultured on 2 x YT containing tetracycline, plasmids were isolated by the rapid isolation method, and direction of tPA023 gene insertion was examined by digestion with Pst I. It was demonstrated that the plasmid having tPA023 gene correctly linked with SV40 promoter in pVY1A ( pVY1AtPA023 ) shows bands of about 3.6 Kbp, about 2.3 Kbp, about 1.5 Kbp ( double ), about 620 bp, about 410 bp, about 80 bp and about 70 bp.

Shown below is the base sequence for the precursor protein, which base sequence constitutes part of the tPA gene.

5' ATGGATGCAATGAAGAGAGGGCTCTGCTGT
GTGCTGCTGCTGTGTGGAGCAGTCTTCGTT
TCGCCCAGCCAGGAAATCCATGCCCGATTC
AGAAGAGGCGCCCGATCTTACCAAGTGATC
TGCAGAGATGAAAAAACGCAGATGATATAC
CAGCAACATCAGTCATGGCTGCGCCCTGTG
CTCAGAAGCAACCGGGTGGAATATTGCTGG

```
                 220              230              240
TGCAACAGTGGCAGGGCACAGTGCCACTCA
                 250              260              270
GTGCCTGTCAAAAGTTGCAGCGAGCCAAGG
                 280              290              300
TGTTTCAACGGGGGCACCTGCCAGCAGGCC
                 310              320              330
CTGAACTTCTCAGATTTCGTGTGCCAGTGC
                 340              350              360
CCCGAAGGATTTGCTGGGAAGTGCTGTGAA
                 370              380              390
ATAGATACCAGGGCCACGTGCTACGAGGAC
                 400              410              420
CAGGGCATCAGCTACAGGGGCACGTGGAGC
                 430              440              450
ACAGCGGAGAGTGGCGCCGAGTGCACCAAC
                 460              470              480
TGGAACAGCAGCGCGTTGGCCCAGAAGCCC
                 490              500              510
TACAGCGGGCGGAGGCCAGACGCCATCAGG
                 520              530              540
CTGGGCCTGGGGAACCACAACTACTGCAGA
                 550              560              570
AACCCAGATCGAGACTCAAAGCCCTGGTGC
                 580              590              600
TACGTCTTTAAGGCGGGGAAGTACAGCTCA
```

```
           610                620                630
GAGTTCTGCAGCACCCCTGCCTGCTCTGAG
           640                650                660
GGAAACAGTGACTGCTACTTTGGGAATGGG
           670                680                690
TCAGCCTACCGTGGCACGCACAGCCTCACC
           700                710                720
GAGTCGGGTGCCTCCTGCCTCCCGTGGAAT
           730                740                750
TCCATGATCCTGATAGGCAAGGTTTACACA
           760                770                780
GCACAGAACCCCAGTGCCCAGGCACTGGGC
           790                800                810
CTGGGCAAACATAATTACTGCCGGAATCCT
           820                830                840
GATGGGGATGCCAAGCCCTGGTGCCACGTG
           850                860                870
CTGAAGAACCGCAGGCTGACGTGGGAGTAC
           880                890                900
TGTGATGTGCCTCCTGCTCCACCTGCGGC
           910                920                930
CTGAGACAGTACAGCCAGCCTCAGTTTCGC
           940                950                960
ATCAAAGGAGGGCTCTTCGCCGACATCGCC
           970                980                990
TCCCACCCCTGGCAGGCTGCCATCTTTGCC
```

```
                1000              1010            1020
AAGCACAGGAGGTCGCCCGGAGAGCGGTTC
                1030              1040            1050
CTGTGCGGGGGCATACTCATCAGCTCCTGC
                1060              1070            1080
TGGATTCTCTGCCGCCCACTGCTTCCAG
                1090              1100            1110
GAGAGGTTTCCGCCCCACCACCTGACGGTG
                1120              1130            1140
ATCTTGGGCAGAACATACCGGGTGGTCCCT
                1150              1160            1170
GGCGAGGAGGAGCAGAAATTTGAAGTCGAA
                1180              1190            1200
AAATACATTGTCCATAAGGAATTCGATGAT
                1210              1220            1230
GACACTTACGACAATGACATTGCGCTGCTG
                1240              1250            1260
CAGCTGAAATCGGATTCGTCCCGCTGTGCC
                1270              1280            1290
CAGGAGAGCAGCGTGGTCCGCACTGTGTGC
                1300              1310            1320
CTTCCCCGGCGGACCTGCAGCTGCCGGAC
                1330              1340            1350
TGGACGGAGTGTGAGCTCTCCGGCTACGGC
                1360              1370            1380
AAGCATGAGGCCTTGTCTCCTTTCTATTCG
```

14

··· ···

```
         1390            1400          1410
GAGCGGCTGAAGGAGGCTCATGTCAGACTG
         1420            1430          1440
TACCCATCCAGCCGCTGCACATCACAACAT
         1450            1460          1470
TTACTTAACAGAACAGTCACCGACAACATG
         1480            1490          1500
CTGTGTGCTGGAGACACTCGGAGCGGCGGG
         1510            1520          1530
CCCCAGGCAAACTTGCACGACGCCTGCCAG
         1540            1550          1560
GGCGATTCGGGAGGCCCCCTGGTGTGTCTG
         1570            1580          1590
AACGATGCCGCATGACTTTGGTGGGCATC
         1600            1610          1620
ATCAGCTGGGGCCTGGGCTGTGGACAGAAG
         1630            1640          1650
GATGTCCCGGGTGTGTACACCAAGGTTACC
         1660            1670          1680
AACTACCTAGACTGGATTCGTGACAACATG


CGACCGTGA   3'
```

Example 7 Construction of pVY1AtPA024

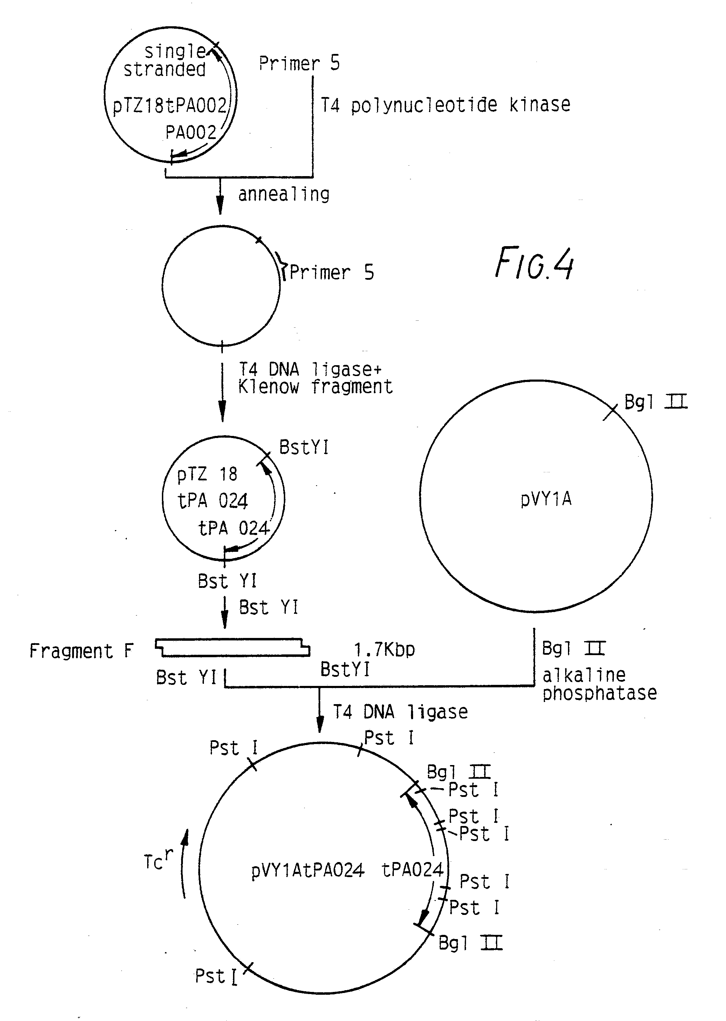

Single-stranded pTZ18tPA002 was subjected to site-specific mutation in the same manner as in Example 5, thus giving tPA024 gene that codes for tPA024 (natural human t-PA with its Ile-86 replaced by Asn). Figure 4 shows the scheme of constructing this mutated gene. Primer 5 employed for the site-specific mutagenesis contains the base sequence as shown below.

5' T GGT ATC ATT TTC ACA GC 3'

The operations were performed in substantially the same manner as in Example 5, except that the colony hybridization for screening was carried out at a temperature of 44°C and that the maximum temperature during washing was 39°C. Finally, base sequence was determined using primer 4 described in Example 4, thereby selecting a plasmid in which the base sequence ATA corresponding to Ile-86 in t-PA had been mutated into AAT corresponding to Asn ( pTZ18tPA024 ).

A fragment of tPA024 gene with a size of about 1.7 Kbp ( fragment F ) was then isolated from plasmid DNA pTZ18tPA024 in the same way as in Example 5.

Fragment F was inserted into expression vector pVY1A in the same manner as in Example 6, and a plasmid containing tPA024 gene correctly linked with SV40 promoter in pVY1A ( pVY1AtPA024 ) was selected. The scheme of construction is shown in Figure 4.

Shown below is the base sequence for the precursor protein, which base sequence constitutes part of the tPA024 gene.

```
                        10            20            30
5' ATGGATGCAATGAAGAGAGGGCTCTGCTGT
                        40            50            60
   GTGCTGCTGCTGTGTGGAGCAGTCTTCGTT
                        70            80            90
   TCGCCCAGCCAGGAAATCCATGCCCGATTC
                        100           110           120
   AGAAGAGGCGCCCGATCTTACCAAGTGATC
                        130           140           150
   TGCAGAGATGAAAAAACGCAGATGATATAC
                        160           170           180
   CAGCAACATCAGTCATGGCTGCGCCCTGTG
                        190           200           210
   CTCAGAAGCAACCGGGTGGAATATTGCTGG
                        220           230           240
   TGCAACAGTGGCAGGGCACAGTGCCACTCA
```

```
          250               260               270
GTGCCTGTCAAAAGTTGCAGCGAGCCAAGG
          280               290               300
TGTTTCAACGGGGGCACCTGCCAGCAGGCC
          310               320               330
CTGTACTTCTCAGATTTCGTGTGCCAGTGC
          340               350               360
CCCGAAGGATTTGCTGGGAAGTGCTGTGAA
          370               380               390
AATGATACCAGGGCCACGTGCTACGAGGAC
          400              410               420
CAGGGCATCAGCTACAGGGGCACGTGGAGC
          430               440               450
ACAGCGGAGAGTGGCGCCGAGTGCACCAAC
          460               470               480
TGGAACAGCAGCGCGTTGGCCCAGAAGCCC
          490               500               510
TACAGCGGGCGGAGGCCAGACGCCATCAGG
          520               530               540
CTGGGCCTGGGGAACCACAACTACTGCAGA
          550               560               570
AACCCAGATCGAGACTCAAAGCCCTGGTGC
          580               590               600
TACGTCTTTAAGGCGGGGAAGTACAGCTCA
          610               620               630
GAGTTCTGCAGCACCCCTGCCTGCTCTGAG
```

17

```
              640              650              660
GGAAACAGTGACTGCTACTTTGGGAATGGG

              670              680              690
TCAGCCTACCGTGGCACGCACAGCCTCACC

              700              710              720
GAGTCGGGTGCCTCCTGCCTCCCGTGGAAT

              730              740              750
TCCATGATCCTGATAGGCAAGGTTTACACA

              760              770              780
GCACAGAACCCCAGTGCCCAGGCACTGGGC

              790              800              810
CTGGGCAAACATAATTACTGCCGGAATCCT

              820              830              840
GATGGGGATGCCAAGCCCTGGTGCCACGTG

              850              860              870
CTGAAGAACCGCAGGCTGACGTGGGAGTAC

              880              890              900
TGTGATGTGCCTCCTGCTCCACCTGCGGC

              910              920              930
CTGAGACAGTACAGCCAGCCTCAGTTTCGC

              940              950              960
ATCAAAGGAGGGCTCTTCGCCGACATCGCC

              970              980              990
TCCCACCCCTGGCAGGCTGCCATCTTTGCC

             1000             1010             1020
AAGCACAGGAGGTCGCCCGGAGAGCGGTTC
```

1030           1040           1050

CTGTGCGGGGGCATACTCATCAGCTCCTGC

1060           1070           1080

TGGATTCTCTCTGCCGCCCACTGCTTCCAG

1090           1100           1110

GAGAGGTTTCCGCCCCACCACCTGACGGTG

1120           1130           1140

ATCTTGGGCAGAACATACCGGGTGGTCCCT

1150           1160           1170

GGCGAGGAGGAGCAGAAATTTGAAGTCGAA

1180           1190           1200

AAATACATTGTCCATAAGGAATTCGATGAT

1210           1220           1230

GACACTTACGACAATGACATTGCGCTGCTG

1240           1250           1260

CAGCTGAAATCGGATTCGTCCCGCTGTGCC

1270           1280           1290

CAGGAGAGCAGCGTGGTCCGCACTGTGTGC

1300           1310           1320

CTTCCCCGGCGGACCTGCAGCTGCCGGAC

1330           1340           1350

TGGACGGAGTGTGAGCTCTCCGGCTACGGC

1360           1370           1380

AAGCATGAGGCCTTGTCTCCTTTCTATTCG

1390           1400           1410

GAGCGGCTGAAGGAGGCTCATGTCAGACTG

```
            1420            1430              1440
      TACCCATCCAGCCGCTGCACATCACAACAT

            1450            1460              1470
      TTACTTAACAGAACAGTCACCGACAACATG

            1480            1490              1500
      CTGTGTGCTGGAGACACTCGGAGCGGCGGG

            1510            1520              1530
      CCCCAGGCAAACTTGCACGACGCCTGCCAG

            1540            1550              1560
      GGCGATTCGGGAGGCCCCCTGGTGTGTCTG

            1570            1580              1590
      AACGATGGCCGCATGACTTTGGTGGGCATC

            1600            1610              1620
      ATCAGCTGGGGCCTGGGCTGTGGACAGAAG

            1630            1640              1650
      GATGTCCCGGGTGTGTACACCAAGGTTACC

            1660            1670              1680
      AACTACCTAGACTGGATTCGTGACAACATG

      CGACCGTGA  3'
```

Example 8 Expression of tPA023 and tPA024 in CHO cells

Plasmids pVY1AtPA023 and pVY1AtPA024 were each transfected in DHFR-deficient CHO cells [ Urlaub, G. and Chasin, L.A., Proc. Natl. Acd. Sci. USA, 77, 4216-4220 ( 1980 )] by the calcium phosphate method [ Graham, F. and van de Eb, A., Virology, 52, 456-467 ( 1973 )]. It was found that transformant clones, S-23t-M10-3(tPA023) and S-24t-M10-1(tPA024), obtained from selection medium [ MEMALPHA(-), GIBCO ] in the presence of methotrexate ( MTX ), produced t-PA activity of 80 U/ml and 100 U/ml respectively ( measured by the fibrin/agarose plate method as described later ). These clones were used for succeeding studies. GIT medium ( Wako Pure Chemical Industry Co., Ltd. ) was used as production medium, with 20 KIU/ml of aprotinin ( SIGMA ) supplemented thereto.

Example 9 Purification of tPA023 and tPA024 from CHO cell culture supernatant

The culture supernatant obtained in Example 8 was partially purified through an anti-t-PA monoclonal antibody affinity column. Anti-t-PA monoclonal antibody-producing hybridoma was obtained conventionally using t-PA derived from human melanoma cells. The hybridoma was inoculated to mice, and the monoclonal antibody developed in the ascites ( subclass: IgG1. ) was recovered and purified by precipitation with ammonium sulfate and through ion-exchange chromatography. The antibody thus obtained was coupled to CNBr-activated Sepharose ( Pharmacia ) by the usual method in a ratio of 5 mg per 1 ml gel.

This antibody Sepharose (10 ml) was mixed with the above culture supernatant ( 4 l ), the mixture was gently shaken overnight at 4°C, and the gel was put in a column ( 1 cm in diameter and 10 cm long ). The gel was washed with 50 ml each of (1) 50mM Tris-HCl buffer ( pH 7.4 ) containing 25 KIU/ml aprotinin ( SIGMA ) and 0.01% (w/v) Tween-80 ( buffer A), (2) buffer A containing 0.5M NaCl, (3) buffer A containing 4M urea, and (4) buffer A containing no aprotinin, in that order. tPA023 and tPA024 bound to the gel were eluted with 0.2M glycine hydrochloride buffer ( pH 2.5 ) containing 0.01% (w/v) Tween-80. The active fractions were collected, and the combined solution was dialyzed against 50 mM acetate buffer ( pH 4 ) containing 100mM NaCl and 0.01% (w/v) Tween-80 and concentrated through an ultrafiltration membrane.

The concentrated dialyzate thus obtained was submitted to succeeding in vitro and in vivo tests. The final product showed an increase in specific acitivity by a factor of 1800 to 2600, indicating 59-71% recovery of t-PA activity ( as measured by the fibrin/agarose plate method ).

Analysis of the active fractions ( tPA023 and tPA024 ) by SDS-electrophoresis and silver staining showed main bands near 66-67 KDa ( kilodaltons ) and 63 KDa in reduced condition, indicating a molecular weight difference of about several thousands of daltons compared with natual human t-PA (main bands : near 62-63 and 58-59KDa).

Furthermore, the gel after electrophoresis was treated with 2.5% (w/v) Triton X-100, put on a fibrin/agarose plate, and subjected to fibrin autoradiography at 37°C. As a result, both tPA023 and tPA024 were found to show dissolved bands near 62-67 KDa ( on the side of higher molecular weight compared with natural human t-PA ).

Shown below are the amino acid sequences of purified tPA023 and tPA024, which are deduced from the above-described base sequences of tPA023 gene and tPA024 gene.

Amino acid sequence of tPA023

|  |  |  |  |  |  |  |  |  |
|---|---|---|---|---|---|---|---|---|
| (H₂N)— Ser | Tyr | Gln | Val | Ile | Cys | Arg | Asp | 8 |
| 9 Glu | Lys | Thr | Gln | Met | Ile | Tyr | Gln | 16 |
| 17 Gln | His | Gln | Ser | Trp | Leu | Arg | Pro | 24 |
| 25 Val | Leu | Arg | Ser | Asn | Arg | Val | Glu | 32 |
| 33 Tyr | Cys | Trp | Cys | Asn | Ser | Gly | Arg | 40 |
| 41 Ala | Gln | Cys | His | Ser | Val | Pro | Val | 48 |
| 49 Lys | Ser | Cys | Ser | Glu | Pro | Arg | Cys | 56 |
| 57 Phe | Asn | Gly | Gly | Thr | Cys | Gln | Gln | 64 |
| 65 Ala | Leu | Asn | Phe | Ser | Asp | Phe | Val | 72 |
| 73 Cys | Gln | Cys | Pro | Glu | Gly | Phe | Ala | 80 |
| 81 Gly | Lys | Cys | Cys | Glu | Ile | Asp | Thr | 88 |
| 89 Arg | Ala | Thr | Cys | Tyr | Glu | Asp | Gln | 96 |
| 97 Gly | Ile | Ser | Tyr | Arg | Gly | Thr | Trp | 104 |

```
105  Ser  Thr  Ala  Glu  Ser  Gly  Ala  Glu  112
113  Cys  Thr  Asn  Trp  Asn  Ser  Ser  Ala  120
121  Leu  Ala  Gln  Lys  Pro  Tyr  Ser  Gly  128
129  Arg  Arg  Pro  Asp  Ala  Ile  Arg  Leu  136
137  Gly  Leu  Gly  Asn  His  Asn  Tyr  Cys  144
145  Arg  Asn  Pro  Asp  Arg  Asp  Ser  Lys  152
153  Pro  Trp  Cys  Tyr  Val  Phe  Lys  Ala  160
161  Gly  Lys  Tyr  Ser  Ser  Glu  Phe  Cys  168
169  Ser  Thr  Pro  Ala  Cys  Ser  Glu  Gly  176
177  Asn  Ser  Asp  Cys  Tyr  Phe  Gly  Asn  184
185  Gly  Ser  Ala  Tyr  Arg  Gly  Thr  His  192
193  Ser  Leu  Thr  Glu  Ser  Gly  Ala  Ser  200
201  Cys  Leu  Pro  Trp  Asn  Ser  Met  Ile  208
209  Leu  Ile  Gly  Lys  Val  Tyr  Thr  Ala  216
217  Gln  Asn  Pro  Ser  Ala  Gln  Ala  Leu  224
225  Gly  Leu  Gly  Lys  His  Asn  Tyr  Cys  232
233  Arg  Asn  Pro  Asp  Gly  Asp  Ala  Lys  240
241  Pro  Trp  Cys  His  Val  Leu  Lys  Asn  248
249  Arg  Arg  Leu  Thr  Trp  Glu  Tyr  Cys  256
257  Asp  Val  Pro  Ser  Cys  Ser  Thr  Cys  264
265  Gly  Leu  Arg  Gln  Tyr  Ser  Gln  Pro  272
273  Gln  Phe  Arg  Ile  Lys  Gly  Gly  Leu  280
281  Phe  Ala  Asp  Ile  Ala  Ser  His  Pro  288
289  Trp  Gln  Ala  Ala  Ile  Phe  Ala  Lys  296
297  His  Arg  Arg  Ser  Pro  Gly  Glu  Arg  304
305  Phe  Leu  Cys  Gly  Gly  Ile  Leu  Ile  312
```

```
313  Ser Ser Cys Trp Ile Leu Ser Ala  320
321  Ala His Cys Phe Gln Glu Arg Phe  328
329  Pro Pro His His Leu Thr Val Ile  336
337  Leu Gly Arg Thr Tyr Arg Val Val  344
345  Pro Gly Glu Glu Glu Gln Lys Phe  352
353  Glu Val Glu Lys Tyr Ile Val His  360
361  Lys Glu Phe Asp Asp Asp Thr Tyr  368
369  Asp Asn Asp Ile Ala Leu Leu Gln  376
377  Leu Lys Ser Asp Ser Ser Arg Cys  384
385  Ala Gln Glu Ser Ser Val Val Arg  392
393  Thr Val Cys Leu Pro Pro Ala Asp  400
401  Leu Gln Leu Pro Asp Trp Thr Glu  408
409  Cys Glu Leu Ser Gly Tyr Gly Lys  416
417  His Glu Ala Leu Ser Pro Phe Tyr  424
425  Ser Glu Arg Leu Lys Glu Ala His  432
433  Val Arg Leu Tyr Pro Ser Ser Arg  440
441  Cys Thr Ser Gln His Leu Leu Asn  448
449  Arg Thr Val Thr Asp Asn Met Leu  456
457  Cys Ala Gly Asp Thr Arg Ser Gly  464
465  Gly Pro Gln Ala Asn Leu His Asp  472
473  Ala Cys Gln Gly Asp Ser Gly Gly  480
481  Pro Leu Val Cys Leu Asn Asp Gly  488
489  Arg Met Thr Leu Val Gly Ile Ile  496
497  Ser Trp Gly Leu Gly Cys Gly Gln  504
505  Lys Asp Val Pro Gly Val Tyr Thr  512
513  Lys Val Thr Asn Tyr Leu Asp Trp  520
521  Ile Arg Asp Asn Met Arg Pro-(COOH)
```

Amino acid sequence of tPA024

```
(H₂N)- Ser  Tyr  Gln  Val  Ile  Cys  Arg  Asp    8
      9  Glu  Lys  Thr  Gln  Met  Ile  Tyr  Gln   16
     17  Gln  His  Gln  Ser  Trp  Leu  Arg  Pro   24
     25  Val  Leu  Arg  Ser  Asn  Arg  Val  Glu   32
     33  Tyr  Cys  Trp  Cys  Asn  Ser  Gly  Arg   40
     41  Ala  Gln  Cys  His  Ser  Val  Pro  Val   48
     49  Lys  Ser  Cys  Ser  Glu  Pro  Arg  Cys   56
     57  Phe  Asn  Gly  Gly  Thr  Cys  Gln  Gln   64
     65  Ala  Leu  Tyr  Phe  Ser  Asp  Phe  Val   72
     73  Cys  Gln  Cys  Pro  Glu  Gly  Phe  Ala   80
     81  Gly  Lys  Cys  Cys  Glu  Asn  Asp  Thr   88
     89  Arg  Ala  Thr  Cys  Tyr  Glu  Asp  Gln   96
     97  Gly  Ile  Ser  Tyr  Arg  Gly  Thr  Trp  104
    105  Ser  Thr  Ala  Glu  Ser  Gly  Ala  Glu  112
    113  Cys  Thr  Asn  Trp  Asn  Ser  Ser  Ala  120
    121  Leu  Ala  Gln  Lys  Pro  Tyr  Ser  Gly  128
    129  Arg  Arg  Pro  Asp  Ala  Ile  Arg  Leu  136
    137  Gly  Leu  Gly  Asn  His  Asn  Tyr  Cys  144
    145  Arg  Asn  Pro  Asp  Arg  Asp  Ser  Lys  152
    153  Pro  Trp  Cys  Tyr  Val  Phe  Lys  Ala  160
    161  Gly  Lys  Tyr  Ser  Ser  Glu  Phe  Cys  168
    169  Ser  Thr  Pro  Ala  Cys  Ser  Glu  Gly  176
    177  Asn  Ser  Asp  Cys  Tyr  Phe  Gly  Asn  184
    185  Gly  Ser  Ala  Tyr  Arg  Gly  Thr  His  192
    193  Ser  Leu  Thr  Glu  Ser  Gly  Ala  Ser  200
    201  Cys  Leu  Pro  Trp  Asn  Ser  Met  Ile  208
```

```
209  Leu Ile Gly Lys Val Tyr Thr Ala  216
217  Gln Asn Pro Ser Ala Gln Ala Leu  224
225  Gly Leu Gly Lys His Asn Tyr Cys  232
233  Arg Asn Pro Asp Gly Asp Ala Lys  240
241  Pro Trp Cys His Val Leu Lys Asn  248
249  Arg Arg Leu Thr Trp Glu Tyr Cys  256
257  Asp Val Pro Ser Cys Ser Thr Cys  264
265  Gly Leu Arg Gln Tyr Ser Gln Pro  272
273  Gln Phe Arg Ile Lys Gly Gly Leu  280
281  Phe Ala Asp Ile Ala Ser His Pro  288
289  Trp Gln Ala Ala Ile Phe Ala Lys  296
297  His Arg Arg Ser Pro Gly Glu Arg  304
305  Phe Leu Cys Gly Gly Ile Leu Ile  312
313  Ser Ser Cys Trp Ile Leu Ser Ala  320
321  Ala His Cys Phe Gln Glu Arg Phe  328
329  Pro Pro His His Leu Thr Val Ile  336
337  Leu Gly Arg Thr Tyr Arg Val Val  344
345  Pro Gly Glu Glu Glu Gln Lys Phe  352
353  Glu Val Glu Lys Tyr Ile Val His  360
361  Lys Glu Phe Asp Asp Asp Thr Tyr  368
369  Asp Asn Asp Ile Ala Leu Leu Gln  376
377  Leu Lys Ser Asp Ser Ser Arg Cys  384
385  Ala Gln Glu Ser Ser Val Val Arg  392
393  Thr Val Cys Leu Pro Pro Ala Asp  400
401  Leu Gln Leu Pro Asp Trp Thr Glu  408
409  Cys Glu Leu Ser Gly Tyr Gly Lys  416
```

```
417  His Glu Ala Leu Ser Pro Phe Tyr  424
425  Ser Glu Arg Leu Lys Glu Ala His  432
433  Val Arg Leu Tyr Pro Ser Ser Arg  440
441  Cys Thr Ser Gln His Leu Leu Asn  448
449  Arg Thr Val Thr Asp Asn Met Leu  456
457  Cys Ala Gly Asp Thr Arg Ser Gly  464
465  Gly Pro Gln Ala Asn Leu His Asp  472
473  Ala Cys Gln Gly Asp Ser Gly Gly  480
481  Pro Leu Val Cys Leu Asn Asp Gly  488
489  Arg Met Thr Leu Val Gly Ile Ile  496
497  Ser Trp Gly Leu Gly Cys Gly Gln  504
505  Lys Asp Val Pro Gly Val Tyr Thr  512
513  Lys Val Thr Asn Tyr Leu Asp Trp  520
521  Ile Arg Asp Asn Met Arg Pro-(COOH)
```

Example 10 Measurement of specific activity of tPA023 and tPA024

The protein content of the partially purified tPA023 and tPA024 was determined by measuring the total protein by the Bradford's method [ Anal. Biochem., 72, 248 ( 1976 )] using bovine serum albumin as standard, and the amount of t-PA antigen was measured by ELISA. ELISA is a sandwich-type method using the monoclonal antibody employed in the above-described antibody column and biotinylated rabbit anti-t-PA antibodies (American Diagnostica Inc. ), in which color was developed by the use of strepavidin-biotinylated horseraddish peroxidase complex ( Amersham Co., Ltd. ) and its substrate ( 3,3',5,5'-tetramethylbenzidine ). As standard t-PA, was used two-chain t-PA derived from human melanoma cells ( American Diagnostica Inc. ).

Fibrinolytic activity was measured by the fibrin/agarose plate method and by the $^{125}$I-labeled fibrin film dissolution method. The former method used an agar-supplemented fibrin plate prepared from 95%-clottable fibrinogen. The latter was performed according to the method of Hoyraerts et al. [ J. Biol. Chem., 257, 2912-2919 ( 1982 )] - a suitable amount of $^{125}$I-labeled fibrinogen ( ICN Biomedical Co., Ltd. ) was added to 1.8μM fibrinogen, the resulting mixture was put on a 96-well microtiter plate ( Nunc ) in an amount of 50 μl/well, followed by drying overnight at 40°C. 1.6 U/ml thrombin ( Mochida Pharmaceutical Co., Ltd. ) was then added in an amount of 100 μl/well, and the system was allowed to stand at 37°C for four hours to effect fibrination(polymerisation). After washing twice with 10mM phosphate buffer containing 0.2% bovine serum albumin (BSA) and 0.9% NaCl, the plate was submitted to activity testing as described below. To each well, were added 50 μl of 200nM plasminogen and 50 μl of standard t-PA, or a novel protein of this invention, in that order, followed by mixing, and the system was held at 37°C for two hours to complete reaction. From each well, was taken 50 μl of reaction mixture, the amount of dissolved $^{125}$I-fibrin was measured using Auto Well Gamma Counter ( Aloca Inc. ), thereby calculating the fibrinolytic activity of the novel proteins of this invention from a standard curve prepared using standard t-PA. The standard t-PA used in this case was t-PA derived from human melanoma cells ( BioScott Inc. ) standardized according to the International t-PA Standard [ Gaffuey and Curtis, Thromb. Haemostas., 53, 134 ( 1985 )].

The specific activity calculated from the activity values measured by the $^{125}$I-fibrin film method and the amount of antigen measured by ELISA was 200,000 U/mg for tPA023, 60,000 U/mg for tPA024 and 890,000 U/mg for natural, human t-PA.

Example 11 Affinity of tPA023 and tPA024 for fibrin and activation of the same by fibrin

Affinity of the two novel proteins for fibrin was examined according to the method of Verheijen et al. [ EMBO J., 5, 3525-3530 ( 1986 )]. To fibrinogen of various concentrations, was added 2 μg/mg of tPA023, tPA024 or natural human t-PA, 1 unit of thrombin was further added, and the resulting mixture was held at room temperature for three minutes to complete reaction. The fibrin clot thus formed was precipitated by centrifugation for eight minutes at 16,000 rpm, and the amount of t-PA left unbound to fibrin in the supernatant was determined by activity measurement by the fibrin/agarose plate method. It was found that the affinity of tPA023 for fibrin is slightly lower compared with natural human t-PA, while tPA024 is distinctly poorer than natural human t-PA in this respect. The following experiment was then carried out to examine the plasminogen activating rates of tPA023 and tPA024 in the presence and absence of fibrin. Using a 96-well microtiter plate, tPA023, tPA024 or natural human t-PA was added to 50mM Tris-HCl buffer ( pH 7.4 ) containing 0.01% Tween-80, 1mM synthetic p-nitroanilide-tripeptide substrate S-2251 ( H-D-Val-Leu-pNA•HCl ), 0.15μM plasmin-free plasminogen and 100 μg/ml of DESAFIBTM ( a soluble substitute for fibrin; American Diagnostica Inc. ) to make the total volume 100 μl in each well. The system was held at 37°C for a definite period, and absorbance at 405 nm ( A405nm ) was measured using Titertech Multiscan. Fibrin-dependent activation was also observed with the two novel proteins of this invention, tPA023 and tPA024, like natural human t-PA. tPA023, in particular, showed higher activation rate ( a factor of 71 in contrast to a factor of 10 to 21 for natural human t-PA ).

Example 12 Analysis on fibrinolytic activity of the proteins of this invention in rabbit blood flow

A pharmacological test using rabbits as test animal revealed that the two novel proteins of this invention, tPA023 and tPA024, both showed a markedly prolonged half life of their enzyme activity compared with natural human t-PA - a half life of about ten minutes for both tPA023 and tPA024, in constrast to a half life of about one to two minutes for natural, human t-PA. In addition, it was noted that tPA023 and tPA024 retained 30% activity even 30 minutes after administration ( with the activity value 30 seconds after administration taken as 100% ), while the activity retention of natural human t-PA was less than 1% 30 minutes after administration. This test was carried out as described below.

Japanese white rabbits weighing 2.5 Kg were used in this experiment. To each test animal was administered, under pentobarbital anesthesia through the peripheral vein of the ear, 0.27 mg/Kg of tPA023 or 0.26 mg/Kg of tPA024 or 0.1 mg/Kg of natural human t-PA. Blood samples ( 2.5 ml each ) were taken from the femoral artery using a catheter at various time intervals ( 0.5 to 60 minutes ) and put in 1/9 volume of 3.8% sodium citrate solution, and low-speed centrifugation was performed within 30 minutes after sampling to separate plasma, which was used for the measurement of t-PA activity in the blood.

Measurement of t-PA activity

After diluting 0.2 ml plasma 1:16 with 3mM glacial acetic acid, the dilution was centrifuged at a low speed, and the separated precipitate was dissolved in a buffer solution containing 20mM Tris-HCl ( pH 7.4 ) and 140mM NaCl ( the same volume as that of original plasma ), giving an euglobulin fraction. The t-PA activity was determined by adding this euglobulin fraction to a fibrin/agarose plate - after incubation of the plate at 37°C for 16 hours, the t-PA activity was observed as plaque (dissolution circle). The fibrin/agarose plate was prepared as described below.

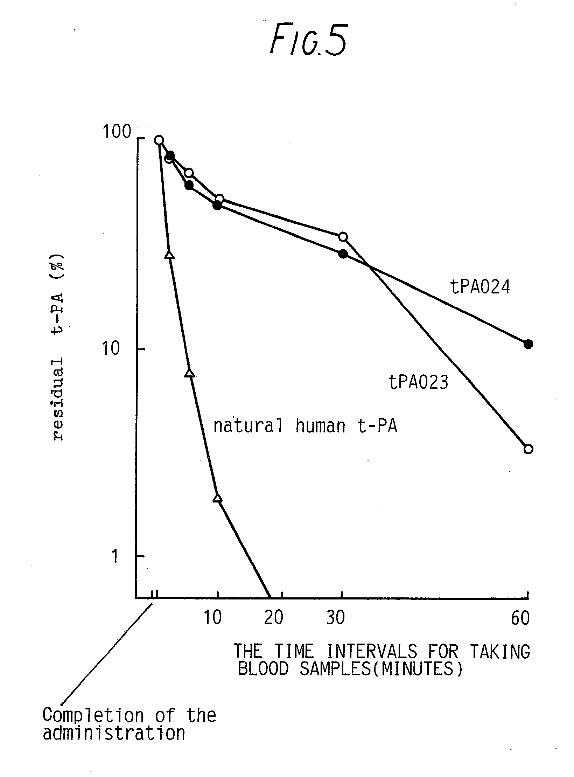

Commercially available fibrinogen ( fraction I of corn ) was used as plasminogen-rich fibrinogen for the preparation of fibrin/agarose plates. The final concentration of plasminogen-rich fibrinogen was 1.5 mg/ml in 20mM Tris-HCl buffer ( pH 7.4 ) containing 130mM NaCl and $10^{-4}$M CaCl$_2$, and the final agarose concentration was 0.75% in said buffer. A plate was prepared by adding 100 μl of thrombin solution ( 40 NIH Unit/ml ) to 10 ml of a fibrinogen/agarose solution. The standard curve for the fibrin/agarose method was prepared using 0.1 to 10,000 U/ml dilutions of the t-PA employed in the animal test. The t-PA activity thus determined was expressed in percentage, using the value of blood sample taken 30 seconds after administration as 100%. The result obtained is summarized in Figure 5.

In the accompanying drawings :

Figure 1 shows a scheme for constructing tPA002 gene and vector pTZ18tPA002 containing said gene ;

Figure 2 shows a scheme for constructing expression vector pVY1A ;

Figure 3 shows the step of mutating tPA002 gene to tPA023 gene and the step of subcloning tPA023 fragment in expression vector pVY1A;

Figure 4 shows the step of mutating tPA002 gene to tPA024 gene and the step of subcloning tPA024 fragment in expression vector pVY1A ; and

Figure 5 compares the clearance rate of the novel proteins of this invention, tPA023 and tPA024, in rabbit blood flow with that of natural human t-PA.

## Claims

1. A thrombolytic protein having an amino acid sequence similar to that of natural human tissue plasminogen activator (natural human t-PA), in which at least one asparagine-linked oligosaccharide chain is attached to a sequence in its epidermal growth factor domain (EGF domain ).

2. A thrombolytic protein having an amino acid sequence similar to that of natural human t-PA, in which Tyr-67 is replaced by Asn-67 with an asparagine-linked oligosaccharide chain attached thereto.

3. A thrombolytic protein having an amino acid sequence similar to that of natural human t-PA, in which Ile-86 is replaced by Asn-86 with an asparagine-linked oligosaccharide chain attached thereto.

4. A DNA molecule that codes for the amino acid sequence of a thrombolytic protein as defined in any of claims 1, 2 and 3.

5. A process for producing proteins which comprises introducing a DNA molecule as defined in claim 4 into an expression vector capable of replicating itself in host cells that allow addition of asparagine-linked oligosaccharide chain; transforming said host cells by said expression vector; culturing the transformants thus obtained under conditions that allow expression of said DNA molecule, thereby producing the corresponding protein; and recovering said protein from the culture liquor.

6. An agent for the treatment of thrombotic disease comprising a therapeutically effective amount of protein as defined in any of claims 1, 2 and 3, and pharmaceutically acceptable carrier.

FIG.1

# FIG.2

FIG.3

FIG.4

# FIG.5

residual t-PA (%)

tPA024

tPA023

natural human t-PA

THE TIME INTERVALS FOR TAKING
BLOOD SAMPLES(MINUTES)

Completion of the
administration

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | THE JOURNAL OF BIOLOGICAL CHEMISTRY, vol. 263, no. 12, April 1988, pages 5948-5954; C.E. MACHAMER et al.: "Influence of new glycosylation sites on expression of the vesicular stomatitis virus G protein at the plasma membrane" * Whole article * | 1 | C 12 N 9/64 C 12 N 15/58 A 61 K 37/547 |
| P,X | WO-A-8 911 531 (GENENTECH, INC.) * Claims 1-13,54 * | 1-6 | |

TECHNICAL FIELDS SEARCHED (Int. Cl.5)

C 12 N

The present search report has been drawn up for all claims

| Place of search | Date of completion of the search | Examiner |
|---|---|---|
| THE HAGUE | 15-03-1990 | HUBER-MACK A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)